# EUROPÄISCHE PATENTSCHRIFT

(11) **EP 0 377 009 B1**
(45) Veröffentlichungstag und Bekanntmachung des Hinweises auf die Patenterteilung: **29.12.1993**
(21) Anmeldenummer: 89906044.6
(22) Anmeldetag: 30.05.1989
(51) Int. Cl.: C07J 1/00, C12P 33/00, C07J 21/00, C07J 41/00

(54) **1$g(b),15$g(a)-DIHYDROXY-1$g(a)-METHYL-5$g(a)-ANDROSTAN-3,17-DION, SEINE HERSTELLUNG UND VERWENDUNG**
1$g(b),15$g(a)-DIHYDROXY-1$g(a)-METHYL-5$g(a)-ANDROSTAN-3,17-DIONE, ITS PREPARATION AND USE
1$g(b),15$g(a)-DIHYDROXY-1$g(a)-METHYLE-5$g(a)-ANDROSTAN-3,17-DIONE, SA FABRICATION ET SON EMPLOI

(30) Priorität: 30.05.1988 DE 3818747
(43) Veröffentlichungstag der Anmeldung: 11.07.1990
(73) Patentinhaber: SCHERING AKTIENGESELLSCHAFT, 13342 Berlin (DE)
(72) Erfinder: PETZOLDT, Karl, D-1000 Berlin 38 (DE); HOFMEISTER, Helmut, D-1000 Berlin 28 (DE)
(86) Internationale Anmeldenummer: DE8900362
(87) Internationale Veröffentlichungsnummer: WO8912064

(56) Entgegenhaltungen:
- DE-A- 3 331 288
- Journal of the Chemical Society, Perkin Transactions I, 1975, London GB, J M Evans et al.: "Microbiological hydroxylation. Part XVI. Incubation of derivatives (mainly acetals) of 5 alpha-androstane ketones with the fungi Calonectria decora, Aspergillus ochraceus, and Rhizopus nigricans", see pages 1356-1360
- Journal of the Chemical Society, Perkin, Transactions I, 1972, London, GB, I M Clark et al.: "Studies in the steroid group. Part LXXXIV. Preparations and reactions of 15-oxygenated androstanes" pages 2765-2770

## Beschreibung

Die Erfindung betrifft das 1β,15α-Dihydroxy-1α-methyl-5α-androstan-3,17-dion der Formel I 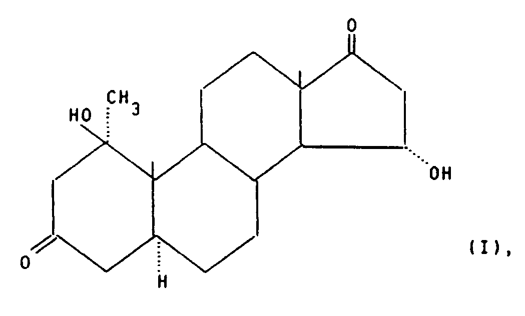
Ferner betrifft die Erfindung ein Verfahren zur Herstellung von 1β,15α-Dihydroxy-1α-methyl-5α-androstan-3,17-dion, welches dadurch gekennzeichnet ist, daß man 1α-Methyl-5α-androstan-3,17-dion der Formel II 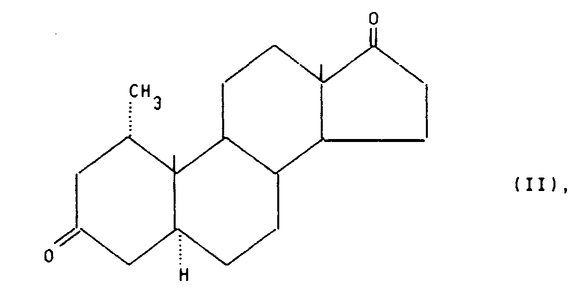
mit einer Kultur eines Mikroorganismusses der Gattung Glomerella oder Fusarium fermentiert.

Unter dem Begriff "Mikroorganismus der Gattung Glomerella oder Fusarium" sollen erfindungsgemäß solche Mikroorganismen verstanden werden, die in anerkannten Hinterlegungsstellen unter den genannten Gattungsnamen deponiert sind und der Fachwelt zur freien Verfügung stehen. Bei den bislang durchgeführten Versuchen wurden 5 Spezies dieser Gattungen auf ihre Fähigkeit zur Durchführung des erfindungsgemäßen Verfahrens untersucht, unter denen sich Glomerella cingulata (ATCC 10534) und Fusarium oxysporum (ATCC 7808) als besonders geeignet erwiesen. Das erfindungsgemäße Verfahren läßt sich nach den vorliegenden Untersuchungen darüberhinaus auch unter Verwendung von Mikroorganismen der Spezies Glomerella fusaroides (ATCC 9552) und Glomerella glycines (ATCC 11871) recht gut durchführen.

Das erfindungsgemäße Verfahren wird unter den gleichen Fermentationsbedingungen durchgeführt, welche man auch bei den bekannten mikrobiologischen Hydroxylierungen von Steroiden mit diesen Mikroorganismen anwendet.

Unter den für diese Mikroorganismen üblicherweise verwendeten Kulturbedingungen werden in einem geeigneten Nährmedium unter Belüften Submerskulturen angezüchtet. Dann setzt man den Kulturen das Substrat (in einem geeigneten Lösungsmittel gelöst oder in emulgierter Form) zu und fermentiert, bis eine maximale Substratumwandlung erreicht ist.

Geeignete Substratlösungsmittel sind beispielsweise Methanol, Ethanol, Glykolmonomethylether, Dimethylformamid oder Dimethylsulfoxyd). Die Emulgierung des Substrats kann beispielsweise bewirkt werden, indem man dieses in mikronisierter Form oder in einem mit Wasser mischbaren Lösungsmittel (wie Methanol, Ethanol, Aceton, Glykolmonomethylether, Dimethylformamid oder Dimethylsulfoxyd) gelöst unter starker Turbulenz in (vorzugsweise entkalktem) Wasser, welches die üblichen Emulgationshilfen enthält, eindüst. Geeignete Emulgationshilfen sind nichtionogene Emulgatoren, wie zum Beispiel Ethylenoxyaddukte oder Fettsäureester von Polyglykolen. Als geeignete Emulgatoren seien die handelsüblichen Netzmittel Tegin®, Tween® und Span® beispielsmäßig genannt.

Die optimale Substratkonzentration, Substratzugabezeit und Fermentationsdauer ist von der Art des verwendeten Mikroorganismus und den Fermentationsbedingungen abhängig. Diese Größen müssen, wie dies bei mikrobiologischen Steroidumwandlungen allgemein erforderlich ist, im Einzelfall durch Vorversuche, wie sie dem Fachmann geläufig sind, ermittelt werden.

Das als Ausgangsmaterial für das erfindungsgemäße Verfahren verwendete 1α-Methyl-5α-androstan-3,17-dion ist vorbekannt (DE-A 25 58 089). Es läßt sich in einfacher Weise durch Oxydation von Mesterolon darstellen.

Für den Fachmann ist es sehr überraschend, daß es mit Hilfe des erfindungsgemäßen Verfahrens möglich ist, das 1β,15α-Dihydroxy-1α-methyl-5α-androstan-3,17-dion in Ausbeuten von ca. 75 % der Theorie darzustellen. Dies deshalb, weil in der Regel gesättigte Steroide nur sehr unbefriedigend hydroxyliert werden, isolierte Ketogruppen meist reduziert werden und eine im Steroidmolekel vorhandene 1-Methylgruppe sich in aller Regel als störend erweist.

Das erfindungsgemäße 1β,15α-Dihydroxy-1α-methyl-5α-androstan-3,17-dion ist ein wertvolles Zwischenprodukt, welches es ermöglicht, zahlreiche pharmakologisch wirksame, in der 15-Position substituierte Steroide auf wesentlich einfachere Weise zu synthetisieren, als dies nach den vorbekannten Verfahren möglich ist.

Derartig pharmakologisch wirksame Steroide sind beispielsweise die aus der EP-A 86730177.2 vorbekannten 15α-Alkyl-1-methyl-1,4-androstadien-3,17-dione und die in der EP-A 88101991.3 vorbeschriebene 1-Methyl-(1-oxyalkyl)-1,4-androstadien-3,17-dione.

Wichtige Zwischenprodukte zur Herstellung dieser Verbindungen sind Androstan-Derivate der allgemeinen Formel III 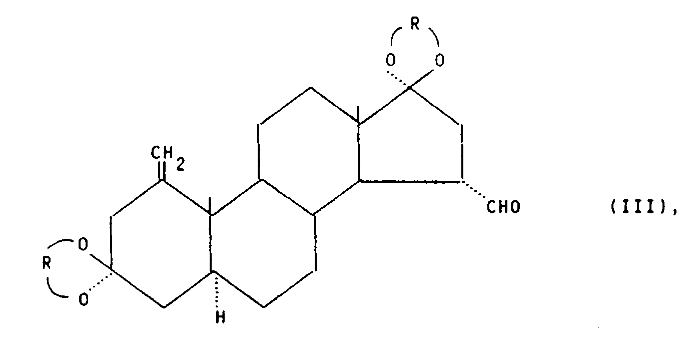
worin
R Jeweils eine Alkylengruppen mit 2 bis 6 Kohlenstoffatomen darstellt. Die vorliegende Erfindung betrifft auch diese Androstan-Derivate der allgemeinen Formel III.

Letztlich betrifft die Erfindung ein Verfahren zur Herstellung dieser Androstan-Derivate, welches dadurch gekennzeichnet ist, daß man die 15α-Hydroxygruppe des gemäß Patentanspruch 1 hergestellten 1β,15α-Dihydroxy-1α-methyl-5α-androstan-3,17-dions gegen eine Cyanidgruppe austauscht, aus dem so dargestellten 15β-Cyan-1β-hydroxy-1α-methyl-5α-androstan-3,17-dion Wasser abspaltet, das erhaltene 15β-Cyan-1-methyl-5α-androst-1-en-3,17-dion mit einem 2 bis 6 Kohlenstoffatome enthaltenden Alkandiol ketalisiert und die gebildeten Cyanoverbindungen der allgemeinen Formel IV 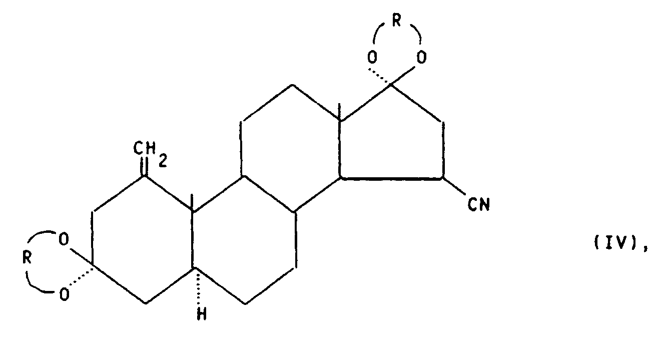
worin
R die oben genannte Bedeutung besitzt partiell reduziert und das erhaltene Gemisch isomerer Aldehyde mittels Basen zu den 15α-Aldehyden der allgemeinen Formel III isomerisiert.

Die Umwandlung des 1β,15α-Dihydroxy-1α-methyl-5α-androstan-3,17-dions in das 15β-Cyan-1β-hydroxy-1α-methyl-5α-androstan-3,17-dion läßt sich in einfacher Weise durchführen, beispielsweise indem man die 15-Hydroxygruppe verestert (zum Beispiel mit Essigsäuranhydrid oder Acetylchlorid) und den Esterrest gegen die Cyanogruppe austauscht.

Dieses ist für den Fachmann überraschend, da bekannt ist, daß es im allgemeinen nicht gelingt, direkt aus Estern organischer Säuren zu den dem Alkoholrest entsprechenden Cyaniden zu gelangen [Methoden der Organischen Chemie (Houben-Weyl, Georg Thieme Verlag, Stuttgart, DE), 4. Auflage, Band VIII, 1952, p 288].

Das erhaltene 15β-Cyan-1β-hydroxy-1α-methyl-5α-androstan-3,17-dion läßt sich erwartungsgemäß in konventioneller Weise dehydratisieren; so beispielsweise durch Einwirkung wasserentziehender Mittel auf diese Verbindung. Ebenso konventionell ist die Ketalisierung des gebildeten 15β-Cyan-1-methyl-5α-androst-1-en-3,17-dions mittels 2 bis 6 Kohlenstoffatome enthaltenden Alkandiolen (wie zum Beispiel 1,2-Ethandiol, 1,3-Propandiol, 2,2-Dimethyl-1,3-propandiol oder 2,3-Butandiol) in Gegenwart wasserentziehender Mittel (wie zum Beispiel Orthoestern) und Säuren oder Lewis-Säuren.

Überraschenderweise ist es nicht erforderlich, die gebildeten 15β-Cyanoverbindungen mittels Basen zu isomerisieren, aus dem Isomerengemisch die 15α-Cyanoverbindungen zu isolieren - was recht aufwendig ist - und diese partiell zu reduzieren, sondern man kann die 15β-Cyanoverbindungen direkt partiell reduzieren (beispielsweise mit komplexen Metallhydriden, wie Diisobutylaluminiumhydrid) und das erhaltene Isomerengemisch von 17α- und 17β-Formylverbindungen mittels Basen überraschenderweise praktisch vollständig zu den gewünschten 17α-Formylverbindungen der allgemeinen Formel III umlagern.

Die weitere Umwandlung der Aldehyde der allgemeinen Formel III in pharmakologisch wirksame Steroide kann in analoger Weise durchgeführt werden, wie diejenige des vorbekannten 17β-Hydroxy-1α-methyl-3α-(2-tetrahydropyranyloxy)-5α-androstan-15α-carboxaldehyds (siehe die oben erwähnten Europäischen Patentanmeldungen).

Die nachfolgenden Ausführungsbeispiele dienen zur näheren Erläuterung des erfindungsgemäßen Verfahrens und der Verwertbarkeit der Verfahrensprodukte.

### A. Ausführungsbeispiele betreffend die erfindungsaemäßen Verfahren

### Beispiel 1

Ein 2-l-Erlenmeyerkolben, der mit 500 ml einer 30 Minuten bei 120° C im Autoklaven sterilisierten Nährlösung enthaltend
3,0 % Glucose
1,0 % Cornsteep liqour
0,2 % Natriumnitrat
0,1 % Kaliumdihydrogenphosphat
0,2 % Dikaliumhydrogenphosphat
0,05 % Magnesiumsulfat-Heptahydrat
0,002 % Eisen(II)-sulfat-Heptahydrat und
0,05 % Kaliumchlorid
beschickt ist, wird mit einer Schrägröhrchen-Kultur des Stammes Glomerella cingulata (ATCC 10534) beimpft und 48 Stunden bei 28° C auf einem Rotationsschüttler (165 Umdrehungen pro Minute) geschüttelt.

Mit 250 ml dieser Anzuchtskultur wird ein 20-l-Vorfermenter beimpft, der mit 15 l eines bei 121° C und 1,1 bar Überdruck sterilisierten Mediums der gleichen Zusammensetzung wie die Anzuchtkultur gefüllt ist. Unter Zugabe von Silicon SH als Antischaummittel wird bei 29° C und 0,7 bar Überdruck unter Belüftung (15 Liter pro Minute) und Rühren (220 Umdrehungen pro Minute) 24 Stunden germiniert.

Danach werden 3 Liter dieser Kultur unter sterilen Bedingungen entnommen und damit ein 75 l Hauptfermenter beimpft, der mit 47 Liter eines wie oben sterilisierten Nährmendiums - enthaltend
1,0 % Glucose
1,0 % Cornsteep liqour
0,2 % Natriumnitrat
0,1 % Kaliumdihydrogenphosphat
0,2 % Dikaliumhydrogenphosphat
0,05 % Magnesiumsulfat-Heptahydrat
0.002 % Eisen(II)-sulfat-Heptahydrat und
0,05 % Kaliumchlorid
beschickt ist.

Nach einer Anwachsphase von 12 Stunden unter Vorfermenterbedingungen wird eine sterilfiltrierte Lösung von 20 g 1α-Methyl-5α-androstan-3,17-dion in 350 ml Dimethylformamid hinzugegeben und weiter gerührt und belüftet. Nach 38 Stunden Kontaktzeit wird die Kulturbrühe zweimal mit je 25 Liter Methylisobutylketon extrahiert, die Extrakte werden vereinigt und im Vakuum zur Trockne eingedampft. Den Rückstand nimmt man in Methanol auf, filtriert das ungelöst gebliebene Siliconöl (Antischaummittel) ab und engt wiederum zur Trockne ein. Der ölig-kristalline Rückstand wird nun aus Aceton umkristallisiert, das Kristallisat mit kaltem Aceton nachgewaschen und im Vakuum-Trockenschrank getrocknet. Man erhält 17,3 g 1β,15α-Dihydroxy-1α-methyl-5α-androstan-3,17-dion vom Schmelzpunkt 227 bis 229° C.

### Beispiel 2

Zwei 2-l-Erlenmeyerkolben, von denen jeder mit 500 ml einer 30 Minuten bei 120° C sterilisierten Nährlösung enthaltend
3,0 % Glucose
1,0 % Cornsteep liqour
0,2 % Natriumnitrat
0,1 % Kaliumdihydrogenphosphat
0,2 % Dikaliumhydrogenphosphat
0,05 % Magnesiumsulfat-Heptahydrat
0,002 % Eisen(II)-sulfat-Heptahydrat und
0,05 % Kaliumchlorid
beschickt ist, werden je mit einer Schrägröhrchen-Kultur des Stammes Fusarium oxysporum (ATCC 7808) beimpft und 48 Stunden bei 28° C auf der Schüttelmaschine

geschüttelt. Mit dem Inhalt der beiden Anzuchtskolben wird ein 30-l-Fermenter beimpft, der mit 19 Liter eines 60 Minuten bei 121° C und 1,1 bar Überdruck sterilisierten Mediums der gleichen Zusammensetzung wie die Anzuchtskultur beschickt ist. Unter Zugabe von Silicon SH als Antischaummittel wird bei 29° C und 0,7 bar Überdruck unter Belüftung (20 Liter pro Minute) und Rühren (220 Umdrehungen pro Minute) germiniert und nach 12 Stunden Anwachszeit das Substrat in Form einer sterilfiltrierten Lösung von 8 g 1α-Methyl-5α-androstan-3,17-dion in 135 ml Dimethylformamid hinzugegeben. Nach weiteren 86 Stunden Rühren und Belüften ist die Umwandlung beendet. Die Kulturbrühe wird nun zweimal mit je 10 Liter Methylisobutylketon extrahiert, die Extrakte vereinigt und im Vakuum zur Trockne eingedampft. Der verbliebene Rückstand wird zur Entfernung des Siliconöls in Methanol gelost und das ungelost gebliebene Antischaummittel mittels eines doppelten Faltenfilters abfiltriert. Die Methanollösung bringt man nach Behandlung mit Aktivkohle wiederum zur Trockne und kristallisiert den Rückstand aus Aceton um. Nach dem Absaugen des Kristallisats und Trocknen im Vakuum-Trockenschrank erhält man 6,1 g 1β,15α-Dihydroxy-1α-methyl-5α-androstan-3,17-dion vom Schmelzpunkt 225-227° C.

### Beispiel 3

a) Zu 10,0 g 1β,15α-Dihydroxy-1α-methyl-5α-androstan-3,17-dion in 40 ml Pyridin werden bei Raumtemperatur 20 ml Essigsäureanhydrid getropft. Nach 2,5 Stunden rührt man das Reaktionsgemisch in Eis/Wasser ein. Das ausgefallene Produkt wird abgesaugt, in Essigester gelöst und mit Wasser neutral gewaschen. Das Rohprodukt wird an Kieselgel mit einem Hexan-Essigester-Gradienten chromatographiert. Es werden 8,6 g 15α-Acetoxy-1β-hydroxy-1α-methyl-5α-androstan-3,17-dion erhalten. Schmelzpunkt 170,0° C.
b) 21,3 g 15α-Acetoxy-1β-hydroxy-1α-methyl-5α-androstan-3,17-dion werden in 146 ml Tetrahydrofuran und 15 ml Wasser unter kräftigem Rühren mit 10,7 g Kaliumcyanid bei 80° C umgesetzt. Nach 20 Stunden wird das Reaktionsgemisch auf die Hälfte eingeengt und in Eis/Wasser eingerührt. Das ausgefallene Produkt wird abgesaugt, in Dichlormethan gelöst und mit Wasser neutral gewaschen. Das Rohprodukt wird an Kieselgel mit einem Methylenchlorid-Aceton-Gradienten chromatographiert. Ausbeute 15β-Cyan-1β-hydroxy-1α-methyl-5α-androstan-3,17-dion. Schmelzpunkt 224° C.
c) 2,0 g 15β-Cyan-1β-hydroxy-1α-methyl-5α-androstan-3,17-dion werden in 20 ml Eisessig mit 6 ml Trifluoressigsäureanhydrid bei Raumtemperatur gerührt. Nach 4 Stunden wird die Lösung in Eis/Wasser gegeben. Das ausgefallene Produkt wird abgesaugt, in Essigester gelöst und mit Wasser gewaschen. Es werden 1,7 g 15β-Cyan-1-methyl-5α-androst-1-en-3,17-dion vom Schmelzpunkt 193° C erhalten.
d) 1,4 g 15β-Cyan-1-methyl-5α-androst-1-en-3,17-dion in 14 ml Dichlormethan weden mit 14 ml 1,2-Ethandiol, 5 ml Trimethylorthoformiat und 25 mg p-Toluolsulfonsäure bei 50° C umgesetzt. Nach 24 Stunden versetzt man die Lösung mit 2 ml Pyridin und engt im Vakuum weitgehend ein. Der Rückstand wird in Eis/Wasser eingerührt. Das ausgefallene Produkt wird abgesaugt, in Essigester gelöst und mit Wasser gewaschen. Nach Chromatographieren des Rohprodukts an Kieselgel mit einem Hexan-Essigester-Gradienten erhält man 1,2 g 3,3;17,17-Bis(ethylendioxy)-1-methylen-5α-androstan-15β-carbonitril vom Schmelzpunkt 229° C.
e) Zu 44,0 g 3,3;17,17-Bis(ethylendioxy)-1-methylen-5α-androstan-15β-carbonitril in 700 ml Toluol werden bei -30° C unter Argon 180 ml einer 1,2 molaren Lösung von Diisobutylaluminium-hydrid in Toluol getropft. Nach 1 Stunde werden bei -30° C etwa 300 ml 10 %ige wässrige Weinsäurelösung langsam zugegeben. Anschließend verdünnt man mit Essigester, gibt weitere 100 ml 10 %ige Weinsäurelosung zu und rührt bei Raumtemperatur. Nach 30 Minuten wäscht man die organische Phase mit Natriumhydrogencarbonat-Lösung und natriumchloridgesättigtem Wasser neutral. Das Rohprodukt wird an Kieselgel mit einem Hexan-Essigester-Gradienten chromatographiert. Es werden 27,5 g 3,3;17,17-Bis(ethylendioxy)-1-methylen-5α-androstan-15α und 15β-carboaldehyd als Isomerengemisch isoliert.
f) 27,5 g 3,3;17,17-Bis(ethylendioxyj-1-methylen-5α-androstan-15α und 15β-carbonitril werden bei Raumtemperatur in 100 ml Methylenchlorid mit 75 ml 1 N methanolischer Kaliumhydroxid-Lösung gerührt. Nach 20 Stunden verdünnt man mit Methylenchlorid und wäscht mit Wasser neutral. Man erhält 27,0 g 3,3;17,17-Bis(ethylendioxyl-1-methylen-5α-androstan-15α-carbaldehyd. Schmelzpunkt 192° C (aus Aceton/Hexan).

### B. Ausführungsbeispiel betreffend die Weiterverarbeitung der Verfahrensprodukte

a) 3,8 g 3,3;17,17-Bis(ethylendioxy)-1-methylen-5α-androstan-15α-carbaldehyd in 46 ml Dimethylformamid werden unter Argon mit 3,0 g Trimethylsulfoniumiodid und 2,3 g Kalium-tert.-butylat bei Raumtemperatur gerührt. Nach 30 Minuten gibt man das Reaktionsgemisch in Eis/Wasser. Das ausgefallene Produkt wird abgesaugt, in Dichlormethan gelöst mit Wasser neutral gewaschen. Es werden 3,5 g 3,3;17,17-Bis(ethylendioxy)-1-methylen-15α-(2-oxiranyl)-5α-androstan als Schaum erhalten.
b) 4,3 g 3,3;17,17-Bis(ethylendioxyl-1-methylen-15α-(2-oxiranyl)-5α-androstan in 100 ml Methanol werden mit 5,8 g Natriummethylat unter Rückfluß gerührt. Das ausgefallene Produkt wird abgesaugt, in Dichlormethan gelöst und mit Wasser neutral gewaschen. Es werden 4,0 g 3,3;17,17-Bis(ethylendioxy)-15α-(1R-hydroxy-2-methoxy-ethyl)-1-methylen-5α-androstan als öliges Produkt erhalten.
c) 3,5 g 3,3;17,17-Bis(ethylendioxy)-15α(1R-hydroxy-2-methoxy-ethyl)-1-methylen-5α-androstan werden in 14 ml Pyridin mit 70 ml Essigsäureanhydrid bei Raumtemperatur umgesetzt. Nach 6 Stunden gibt man die Lösung in Eis/Wasser. Das ausgefallene Produkt wird abgesaugt, in Dichlormethan gelöst und mit Wasser gewaschen. Es werden 3,1 g 15α-(1R-Acetoxy-2-methoxy-ethyl)-3,3;17,17-bis(ethylendioxy)-1-methylen-5α-androstan als Schaum erhalten.
d) 2,9 g 15α-(1R-Acetoxy-2-methoxy-ethyl)-3,3;17,17-bis(ethylendioxy)-1-methylen-5α-androstan in 30 ml Methanol und 6 ml Wasser werden mit 2,9 g Oxalsäure unter Rückfluß gerührt. Nach 1 Stunde wird das Reaktionsgemisch im Vakuum eingeengt und in Eis/Wasser eingerührt. Das ausgefallene Produkt wird abgesaugt und mit Wasser neutral gewaschen. Es werden 1,6 g 15α-(1R-Acetoxy-2-methoxy-ethyl)-1-methyl-5α-androst-1-en-3,17-dion vom Schmelzpunkt 190° C erhalten.
e) 1,2 g 15α-(1R-Acetoxy-2-methoxy-ethyl)1-methyl-5α-androst-1-en-3,17-dion in 40 ml Dioxan werden bei 80° C mit 1,2 g 2,3-Dichlor-5,6-dicyan-p-benzochinon gerührt. Nach 7 Stunden läßt man das Gemisch abkühlen, filtriert, verdünnt das Filtrat mit Essigester und wäscht mehrmals mit Natriumhydrogencarbonat-Lösung und Wasser. Das Rohprodukt wird an Kieselgel mit einem Methylenchlorid-Aceton-Gradienten chromatographiert. Ausbeute: 780 mg 15α-(1R-Acetoxy-2-methoxy-ethyl)-1-methyl-1,4-androstadien-3,17-dion. Schmelzpunkt 184° C (aus Aceton/Isopropylether).

Die so dargestellte Verbindung ist pharmakologisch wirksam. Pharmazeutische Präparate die diese Verbindung enthalten eignen sich unter anderem zur Behandlung östrogeninduzierter oder stimmulierter Tumoren (Mamakarzinom, Prostatahyperplasie) und zur Beeinflussung der Fertilität (EP-A 88 101 991.3).

## Patentansprüche

1. 1β,15α-Dihydroxy-1α-methyl-5α-androstan-3,17-dion der Formel I

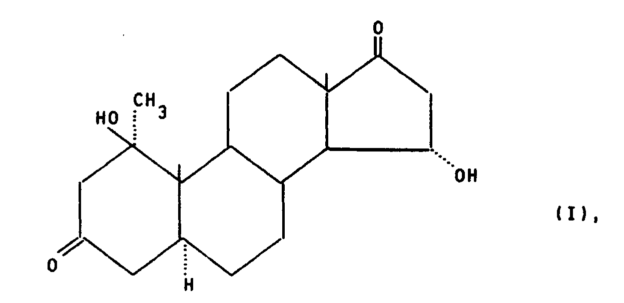

2. Verfahren zur Herstellung von 1β,15α-Dihydroxy-1α-methyl-5α-androstan-3,17-dion, dadurch gekennzeichnet, daß man 1α-Methyl-5α-androstan-3,17-dion der Formel II 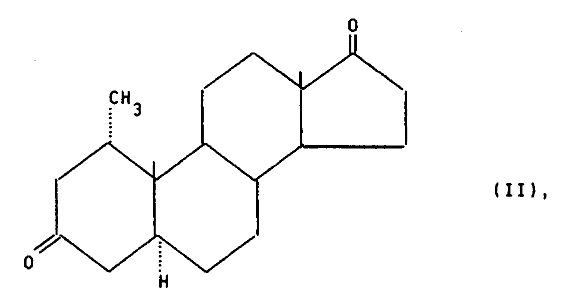 mit einer Kultur eines Mikroorganismusses der Gattung Glomerella oder Fusarium fermentiert.

3. Androstan-Derivate der allgemeinen Formel III 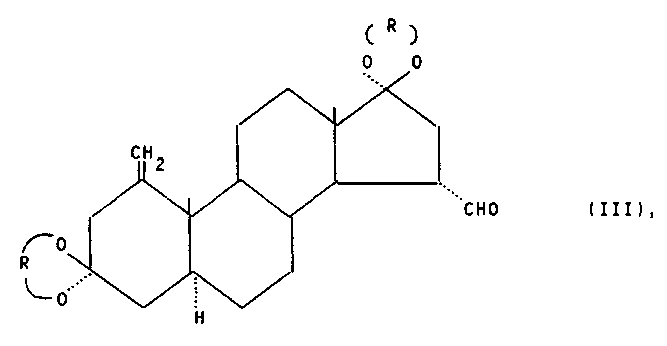 worin
R jeweils eine Alkylengruppen mit 2 bis 6 Kohlenstoffatomen darstellt.

4. Verfahren zur Herstellung der Androstan-Derivate der Formel III gemäss Anspruch 3, welches dadurch gekennzeichnet ist, daß man die 15α-Hydroxygruppe des gemäß Patentanspruch 1 hergestellten 1β,15α-Dihydroxy1α-methyl-5α-androstan-3,17-dions gegen eine Cyanidgruppe austauscht, aus dem so dargestellten 15β-Cyan-1β-hydroxy-1α-methyl-5α-androstan-3,17-dion Wasser abspaltet, das erhaltene 15β-Cyan-1-methyl-5α-androst-1-en-3,17-dion mit einem 2 bis 6 Kohlenstoffatome enthaltenden Alkandiol ketalisiert und die gebildeten Cyanoverbindungen der allgemeinen Formel IV 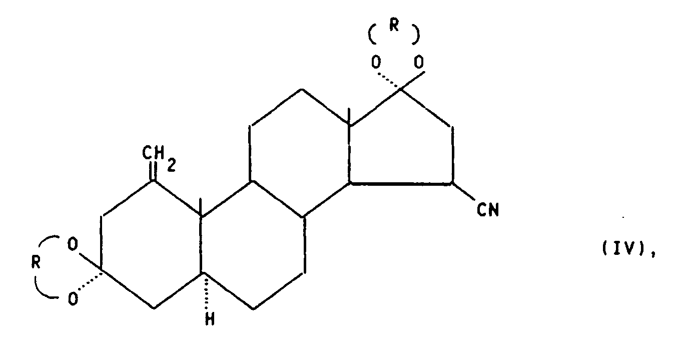 worin
R die oben genannte Bedeutung besitzt partiell reduziert und das erhaltene Gemisch isomerer Aldehyde mittels Basen zu den 15α-Aldehyden der allgemeinen Formel III isomerisiert.

## Claims

1. 1β,15α-dihydroxy-1α-methyl-5α-androstane-3,17-dione of the formula I

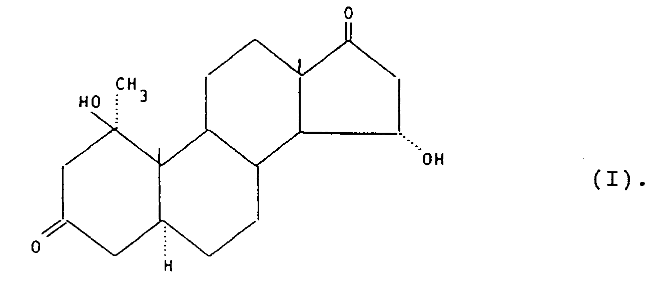

2. Process for the preparation of 1β,15α-dihydroxy-1α-methyl-5α-androstane-3,17-dione, characterised in that 1α-methyl-5α-androstane-3,17-dione of the formula II 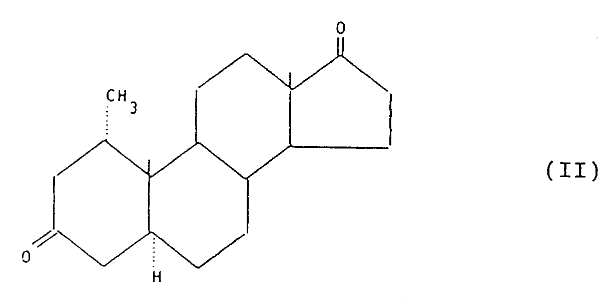 is fermented with a culture of a micro-organism of the genus Glomerella or Fusarium.

3. Androstane derivatives of the general formula III 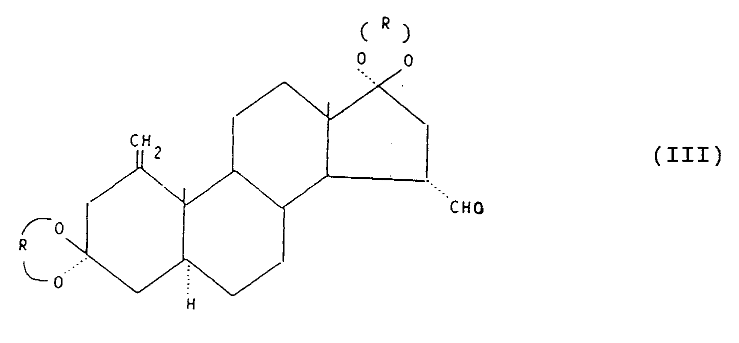 wherein
each R represents an alkylene group containing from 2 to 6 carbon atoms.

4. A process for the preparation of the androstane derivatives of the formula III according to claim 3, which is characterised in that the 15α-hydroxy group of the 1β,15α-dihydroxy-1α-methyl-5α-androstane-3,17-dione prepared in accordance with patent claim 1 is replaced by a cyanide group, water is removed from the 15β-cyano-1β-hydroxy-1α-methyl-5α-androstane-3,17-dione so prepared, the 15β-cyano-1-methyl-5α-androst-1-ene-3,17-dione obtained is ketalised with an alkanediol containing from 2 to 6 carbon atoms and the resulting cyano compounds of the general formula IV 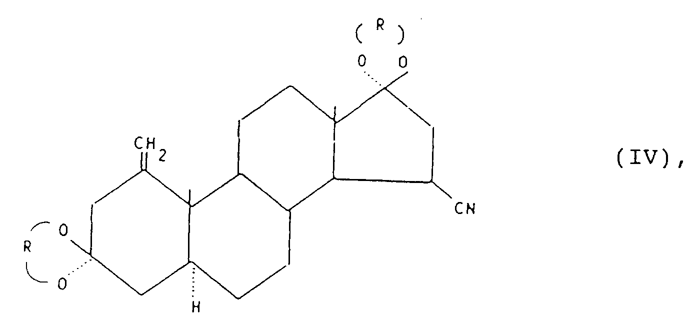 wherein
R is as defined above, are partially reduced and the resulting mixture of isomeric aldehydes is isomerised by means of bases to form the 15α-aldehydes of the general formula III.

## Revendications

1. 1β,15α-Dihydroxy-1α-méthyl-5α-androstane-3,17-dione de formule I ci-dessous :

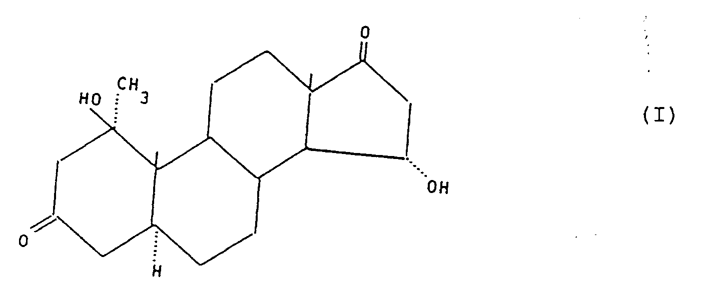

2. Procédé de préparation du composé de la revendication 1, procédé caractérisé en ce que l'on soumet à une fermentation la 1α-méthyl-5α-androstane-3,17-dione de formule II : 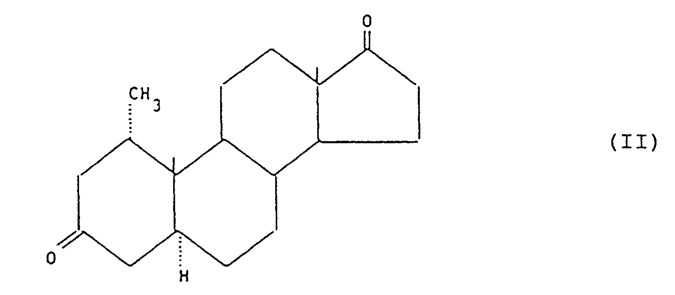 avec une culture d'un microorganisme du genre *Glomerella* ou *Fusarium*.

3. Dérivés d'androstane de formule générale III : 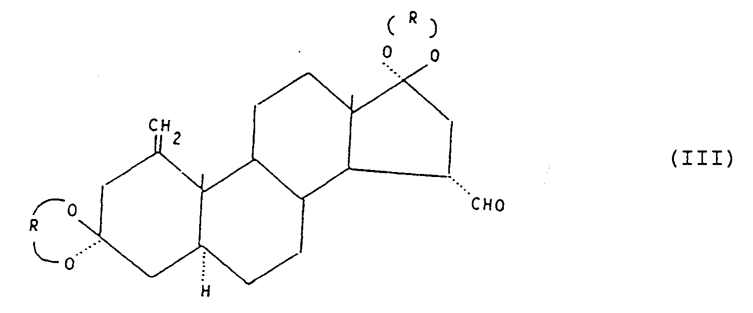 R représentant un alkylène pouvant avoir de 2 à 6 atomes de carbone.

4. Procédé de préparation des dérivés d'androstane de formule III selon la revendication 3, procédé caractérisé en ce que l'on remplace par un groupe cyanure le groupe 15α-hydroxy de la 1β,15α-dihydroxy-1α-méthyl-5α-androstane-3,17-dione obtenue selon la revendication 1, de la 15β-cyano-1β-hydroxy-1α-méthyl-5α-androstane-3,17-dione ainsi formée on élimine une molécule d'eau puis on cétalyse la 15β-cyano-1-méthyl-5α-androst-1-ène-3,17-dione obtenue avec un alcane-diol ayant de 2 à 6 atomes de carbone, on réduit ensuite partiellement les composés cyanés formés de formule IV : 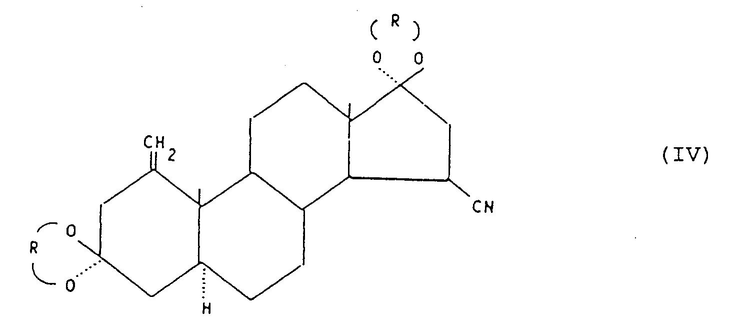 R ayant la signification indiquée à la revendication 3, et on isomérise enfin le mélange obtenu d'aldéhydes isomères avec des bases pour obtenir les 15α-aldéhydes de formule générale III.
